# EUROPEAN PATENT APPLICATION

(11) **EP 2 402 358 A2**
(43) Date of publication of application: **04.01.2012**
(21) Application number: 10172853.3
(22) Date of filing: 15.08.2010
(51) Int. Cl.: C07H 19/073, A61K 31/7068, A61P 31/18

(54) **2',3'-Didehydro-3'-deoxy-4'-ethynylothymidine derivatives for treatment infections caused by human immunodeficiency virus (HIV) multidrug resistant strains, method of their synthesis and pharmaceutically acceptable forms of drugs**

(30) Priority: 30.06.2010 PL 39169510
(71) Applicant: Instytut Biochemii I Biofizyki Pan, 02-106 Warszawa (PL)
(72) Inventor: ZIEMKOWSKI, Przemys?aw, 03-130 Warszawa, Warszawa (PL); MIAZGA, Agnieszka, 37-400, Nisko (PL); KULIKOWSKI, Tadeusz, 02-116, Warszawa (PL); LOUVEL, Severine, F-68220, Hagenthal le haut (FR); KLIMKAIT, Thomas, D-70539, Loerrach (DE); VIDAL, Vincent, F-68300, Saint-Louis (FR)
(74) Representative: Brodowska, Iwona

(57) **Abstract**

4'-Ethynylostavudine derivatives according to the invention substituted at 4'-ethynylostavudine 5'-O-position with 12-tetradodecanoyl, 12-bromododecanoyl, 12-methoxydodecanoyl, 12-ethylothiododecanoyl, or 12-azidododecanoyl group (represented by the symbols **WA37, WA38, WA40, WA42, WA39**) in deClPhR™ cells exert very high antiviral activity against the wild type HIV-1 strain, as well as against its drug and multidrug resistant strains, without any citotoxicty at the therapeutic concentrations (CC₅₀ >200 µM).

These compounds, because of their very low toxicity may be applied at all phases of AIDS, even at the final phase when the T4 lymphocytes level in patients drops down below 200/µL of peripheral blood.

The 2',3'- didehydro-3'-deoxy-4'-ethynylothymidine derivatives, according to the invention, are synthesized by the transformation of the known compound 4'-ethynylostavudine (**WA32,** wherein R represents hydrogen).

## Description

### Technical Field

The present invention relates to new 5'-O-ester derivatives of 2',3'-didehydro-3'-deoxy-4'-ethynylothymidine (4'-ethynylostavudine, 4'*E-*d4T) showing high activity and selectivity against human immunodeficiency virus (HIV-1) including its drug and multidrug resistant (MDR) strains, represented by the general **Formula 1**, wherein **R** represents 12-tetradodecanoyl, 12-bromododecanoyl, 12-methoxydodecanoyl, 12-ethylothiododecanoyl, or 12-azidododecanoyl group, method of their synthesis and containing them pharmaceutical agents.

According to the invention new compounds are pro-drugs of the selective HIV-1 retrovirus inhibitor, 4'-ethynylostavudine (4'-*E-*d4T). The pro-drugs need intracellular hydrolytic cleavage yielding 4'-*E*d4T, followed by its intracellular activation - phosphorylation with host kinases yielding its nucleoside - 5'-triphosphate.

### Background Art

According to current knowledge six 5'-O-myristoil derivatives of 2',3'-dideoxy-3'-fluorothymidine (FLT-derivatives) are known so far and they belong to the most potent HIV inhibitors in various T cells (Parang et al., Nucleosides Nucleotides, 1998, 17, 987-1008), significantly more potent than the standard drug AZT. Unfortunately, FLT released in cells is more toxic towards non-infected lymphocytes than AZT (Balzarini J., Baba M., Pauwels R., Herdewijn P., De Clercq E. Biochem. Pharmacol. 1988, 37, 2847-2856). Clinical evaluations have shown that trombocytopoenia and anaemia are the toxic effects, which limit the drug dose (Balzarini J., Baba M., Pauwels R., Herdewijn P., De Clercq E. Biochem. Pharmacol. 1988, 37, 2847-2856), and for this reason further clinical evaluations have been stopped. Above adverse side effects induced the Inventors to look for new compounds among derivatives of 4'-ethynylostavudine analogs class.

The Inventors unexpectedly discovered that the synthesis of new series of 5'-O-substituted 4'-ethynylostavudine derivatives give compounds with properties of pro-drugs exerting very potent and selective anti-HIV-1 activity, without any toxicity at the therapeutic concentrations.

### Summary of invention

2',3'-Didehydro-3'-deoxy-4'-ethynylothymidine derivatives for treatment infections with human immunodeficiency virus (HIV-1), including its drug and especially multidrug resistant strains are 4'-ethynylostavudine 5'-O-esters represented by the general **Formula 1,** wherein **R** represents: 12-tetradodecanoyl, 12-bromododecanoyl, 12-methoxydodecanoyl, 12-ethylothiododecanoyl, or 12-azidododecanoyl group.

The derivatives according to the invention represented by the general **Formula 1,** wherein **R** represents:
- 12-tetradodecanoyl group for the compound **WA37,**
- 12-bromododecanoyl group for the compound **WA38,**
- 12-methoxydodecanoyl group for the compound **WA40,**
- 12-ethylothiododecanoyl for the compound **WA42,** and
- 12-azidododecanoyl group for the compound **WA39.**

Particularly useful 4'-ethynylostavudine derivatives are compounds represented by the **Formula 1**, wherein **R** represents: 12-tetradodecanoyl, 12-bromododecanoyl, and 12-azidododecanoyl group, that is the compounds represented by the symbols **WA37, WA38,** and **WA39** respectively showing resistance factor (Rf) values better than that of 4'-ethynylostavudine.

The Inventors unexpectedly discovered also that according to the invention new 4'-ethynylostavudine analogues substituted at 5'-O position of ethynylostavudine moiety with 12-tetradodecanoyl, 12-bromododecanoyl, 12-methoxydodecanoyl, 12-ethylothiododecanoyl, or 12-azidododecanoyl group (represented by the symbols **WA37, WA38, WA40, WA42, WA39)** exerted very high antiviral activity in deClPhR™ cells against wild type HIV-1, its drug, as well as multidrug resistant strains, without any citotoxicity at therapeutic concentrations (CC₅₀ >200 mmM) **(Table 1).**

Because of very low toxicity, the compounds of above type may be applied at all phases of AIDS development, also at the final phase, that is when the amount of T4 lymphocytes in peripheral blood of patients drops down below 200/mmL.

2',3'-Didehydro-3'-deoxy-4'-ethynylothymidine derivatives according to the invention for treating infections with human immunodeficiency virus (HIV-1) including its drug and multidrug resistant strains - 4'-ethynylostavudine 5'-O-esters represented by the general **Formula 1,** wherein **R** represents 12-tetradodecanoyl, 12-bromododecanoyl, 12-methoxydodecanoyl, 12-ethylothiododecanoyl, or 12-azidododecanoyl group are synthesized by the transformation of known compound, 4'-ethynylostavudine **(WA32,** wherein **R** represents hydrogen, **Table 1).**

The method of synthesis of 5'-O-substituted 4'-ethynylostavudine derivatives, represented by the general **Formula 1**, wherein **R** represents 12-tetradodecanoyl, 12-bromododecanoyl, 12-methoxydodecanoyl, or 12-ethylothiododecanoyl group (represented by the symbols **WA37, WA38, WA40,** and **WA42** respectively) according to the invention comprises treating the compound **WA32** represented by the **Formula 1,** wherein **R** represents hydrogen, in dichloromethane in the presence of dimethyloaminopyridine for one hour at room temperature with a respective acid halogen represented by the formula **RX,** wherein **R** represents as above and **X** represents halogen **(Scheme 1)**.

The method of synthesis of 5'-O-substituted 4'-ethynylostavudine derivative represented by the **Formula 1,** wherein **R** represents 12-azidododecanoyl group **(WA39)** according to the invention, comprises treating the compound **WA38** with sodium azide in dimethyloformamide at room temperature.

This invention includes also pharmaceutically acceptable form of drug, which contains known pharmaceutically acceptable media and auxiliary materials, according to the invention, and as an active component contains the derivatives represented by the general **Formula 1,** wherein **R** represents 12-tetradodecanoyl, 12-bromododecanoyl, 12-methoxydodecanoyl, 12-ethylothiododecanoyl, and/or 12-azidododecanoyl group.

Pharmaceutically acceptable form of drug showing 4'-ethynylostavudine pro-drug character, exerting high activity against human immunodeficiency virus (HIV-1), and low citotoxicity, contains known pharmaceutically acceptable media and auxiliary substances, and its active component is the compound or a mixture of the compounds belonging to the group, which includes the compounds represented by the general **Formula 1,** wherein **R** represents 12-tetradodecanoyl, 12-bromododecanoyl, 12-methoxydodecanoyl, 12-ethylothiododecanoyl, or 12-azidododecanoyl group, which are the compounds represented by the symbols **WA37, WA38, WA40, WA42, WA39,** which are hydrolyzed in a cell with esterases yielding the compound represented by the symbol **WA32.**

The examples are presented below which illustrate the invention and do not limit its scope.

**Example** I

**General method for the synthesis of 5'-O-myristoylated derivatives of 2',3'-dideoxy-2',3'-didehydro-4'-ethynylthymidine**

To the solution of the appropriate fatty acid (1 mmol) in dry dichloromethane (20 ml) oxalyl chloride (0.2 mL, 2.1 mmol) was added and the mixture was stirred 2 h at room temperature and evaporated to dryness *in vacuo.* Dry residual oil was dissolved in 10 mL of dry dichloromethane and the solution was added dropwise to an ice-cold solution of 4'-ethynylstavudine (248 mg, 1 mmol) and 4-dimethylaminopyridine (DMAP) (176 mg, 1.4 mmol) in dry dichloromethane (10 mL). This solution was stirred at 0°C for 20 min and then at room temperature for 2 h. The mixture was washed with saturated aqueous sodium bicarbonate (2 x 50 mL) and with water (2 x 50 mL). The organic layer was dried with Na₂SO₄ and the solvent was removed *in vacuo.* The residue was purified by silica gel chromatography using dichloromethane/methanol (98:2, v/v) as eluent to yield the respective products **WA38, WA40, WA42.**

1-(5-O-(12-bromododecanoyl)-2,3-dideoxy-2,3-didehydro-4-ethynyl-bb-D-ri bofuranosyl)-thymine **(WA38)**

Yield: 325 mg; TLC (silica gel) R_{f} (CHCl₃:MeOH 97:3) 0.57; UV ^{λ}max (MeOH) 265 nm; MS (M+H)⁺ 510.

1-(2,3-dideoxy-2,3-didehydro-4-ethynyl-5-O-(12-methoxydodecanoyl)-bb-D -ribofuranosy)-thymine **(WA40)**

Yield: 230 mg, 50%; TLC (silica gel) R_{f} (CHCl₃:MeOH 97:3) 0.48; UV λₘₐₓ (MeOH) 265 nm; MS (M+H)⁺ 461.

1-(2,3-dideoxy-2,3-didehydro-5-O-(11-thioethylundecanoyl)-4-ethynyl)-bb-D-ribofuranosyl)thymine **(WA42)**

Yield: 214 mg, 45%; TLC (silica gel) R_{f} (CHCl₃:MeOH 97:3) 0.58; UV ^{λ}max (MeOH) 265 nm; MS (M+H)⁺ 477.

**Example II**

1-(2,3-dideoxy-2,3-didehydro-4-ethynyl-5-O-tetradodecanoyl-bb-D-ribofura nosyl)-thymine **(WA37)**

Myristoyl chloride (0.27 mL, 1 mmol) was added dropwise to the ico-cold mixture of 4'-ethynylstavudine (248 mg, 1 mmol) and 4-dimethylaminopyridine (176 mg, 1.4 mmol) in dry dichloromethane (20 mL). This solution was stirred at 0°C for 1 h, and then at room temperature for 2 h. The mixture was washed with saturated aqueous sodium bicarbonate (2 x 50 mL) and with water (2 x 50 mL). The organic layer was dried with Na₂SO₄ and the solvent was removed *in vacuo.* The residue was purified by silica gel chromatography using dichloromethane/methanol (98:2, v/v) as eluent to yield **WA37** (323 mg, 66%); TLC (silica gel) R_{f} (CHCl₃:MeOH 97:3) 0.58; UV λₘₐₓ (MeOH) 265 nm; MS (M+H)⁺ 459.

**Example III**

1-(5-O-(12-azidododecanoyl)-2,3-dideoxy-2,3-didehydro-4-ethynyl-bb-D-ri bofuranosyl)-thymine **(WA39)**

The solution of 1-(5-O-(12-bromododecanoyl)-2,3-dideoxy-2,3-didehydro-4-ethynyl-bb-D-ri bofuranosyl)-thymine (254 mg, 0.5 mmol) and sodium azide (46 mg, 0.7 mmol) in dimethylformamide (2.5 mL) were stirred for 3 h at room temperature. The solvent was then removed in vacuo and the residue was dissolved in chloroform (50 mL) and extracted with water (2 x 50mL). The organic layer was dried over Na₂SO₄ and evaporated to dryness in vacuo. The oil residue was purified by silica gel chromatography using dichloromethane/methanol (98:2, v/v) as eluent to yield **WA39** (195 mg, 83%); TLC (silica gel) R_{f} (CHCl₃:MeOH 97:3) 0.56; UV λₘₐₓ (MeOH) 265 nm; MS (M+H)⁺ 472.

**Example IV**

The antiviral activity (EC₅₀) and cytotoxicity (CC₅₀) of the derivatives obtained as above (Examples I-III) were investigated in the deClPhR™ cell system with use of the following methods.

*Determination of antiviral activity*

Fully infectious virus HIV-1 was produced from cloned proviral DNA reference coding either for wild-type HIV-1 or from drug-resistant virus following transfection of proviral DNA into recipient mammalian cells. Subsequent to viral protein expression and particle formation, culture supernatant was harvested and used to infect an indicator cell line. This latter cell line expresses all necessary receptors and co-receptors for HIV infection as well as the bacterial gene LacZ coding for β-galactosidase under the control of the HIV-1 long terminal repeat. In this system, amount of β-galactosidase is directly proportional to the extent of viral replication. Activity of β-galactosidase is assayed in presence of a colorless substrate which upon enzymatic conversion yields a yellow product absorbing at 405 nm. Next, variation in colorimetric readout in presence of test compounds was translated in percent viral inhibition following normalization according to absorbance values of positive (presence of anti-HIV drug) and negative (untreated) control wells included in each 96 well-plate. For determining effective concentration 50 % (EC₅₀), test compounds were assayed across a range of concentrations. Percent inhibition data in function of drug concentration were processed by a statistical curve fitting software yielding a modeled curve from which EC₅₀ was extrapolated.

*Determination of cytotoxicity: Alamar Blue*™ *Cell Viability Assay*

The Alamar Blue™ Cell Viability Assay (Invitrogen) is a fluorimetric metod for determining the number of viable cells. The Alamar Blue Reagent contains the cell permeable non-fluorescent blue dye resazurin, which is converted to the pink and fluorescent dye resorufin in response to the action of reductases located in the cytosol, mitochondria and endoplasmic reticulum. Therefore, only living cells will convert resazurin into resorufin. Assays are performed by adding 20 µL of the Alamar Blue Reagent directly to culture wells followed by a 6-hour incubation at 37°C. The fluorescent signal is monitored using 545 nm excitation wavelength and 590 nm emission wavelength. The quantity of resofurin product as measured by fluorescence is directly proportional to the number of living cells in culture, what allows to calculate CC₅₀ value.

**Table 1**. Anti-HIV-1 activity (EC₅₀), resistance factor (Rf)* and cytotoxicity (CC₅₀) of mirystoylated derivatives of 4'-ethynylostavudine (4'-*E*-d4T) in deClPhR™ cells

**Table 1**

| Compound | HIV-1 virus strain | | | Cytotoxicity |
|---|---|---|---|---|
| | Wild type** | Resistant 7005695 | Resistant QCR8 | PNL4-3 |
| | EC₅₀ (nM) | Rf* | Rf* | CC₅₀ (nM) |
| 4'-*E*-d4T (WA32) | 90 | 17.3 | 1.4 | > 200 000 |
| WA37 | 54 | 23.7 | 1.3 | > 200 000 |
| WA38 | 130 | 10.4 | 1 | > 200 000 |
| WA39 | 73 | 12.2 | 0.8 | > 200 000 |
| WA40 | 48 | 17.4 | 2.3 | > 200 000 |
| WA42 | 43 | 16.1 | 2.3 | > 200 000 |

Resistance factor Rf=EC₅₀ mutant / EC₅₀ wt virus

** NL4-3 cytotropic strain (CXCR4 - tropic)

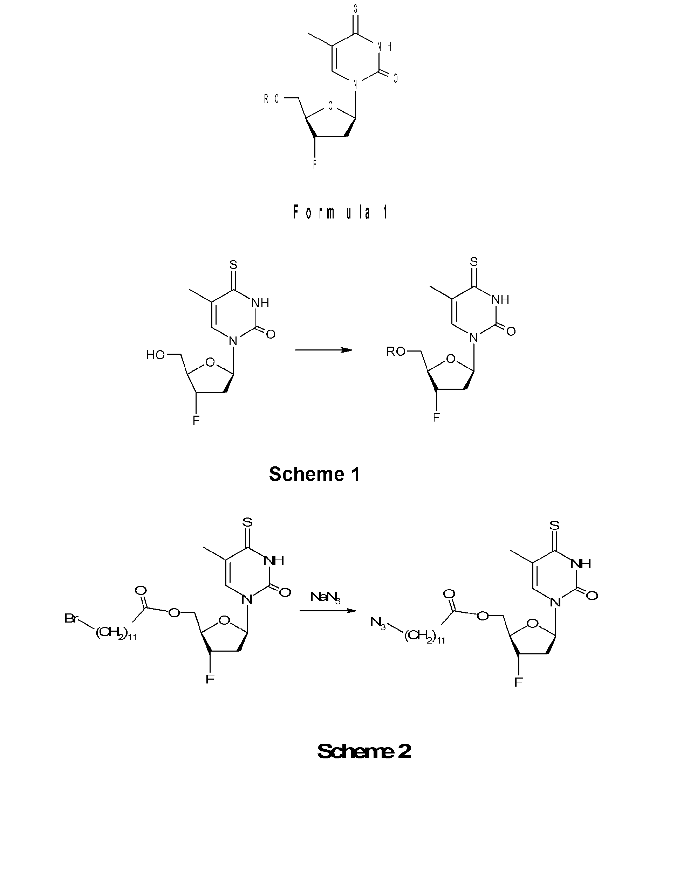

## Claims

1. 2',3'-Didehydro-3'-deoxy-4'-ethynylothymidine derivatives for treatment infections caused by human immunodeficiency virus (HIV) multidrug resistant strains - 5'-O-esters of 4'-ethynylostavudine represented by the general **Formula 1,** wherein **R** represents 12-tetradodecanoyl, 12-bromododecanoyl, 12-methoxydodecanoyl, 12-ethylothiododecanoyl, or 12-azidododecanoyl group.

2. The derivatives according to the claim 1 represented by the general **Formula 1,** wherein **R** represents for the compound **WA37** 12-tetradodecanoyl, for the compound **WA38** 12-bromododecanoyl, for the compound **WA40** 12-methoxydodecanoyl, for the compound **WA42** 12-ethylothiododecanoyl, and for the compound **WA39** 12-azidododecanoyl group.

3. The derivatives according to the claim 1 represented by the general **Formula 1,** wherein **R** represents 12-tetradodecanoyl, 12-bromododecanoyl, 12-methoxydodecanoyl, 12-ethylothiododecanoyl, or 12-azidododecanoyl group.

4. The method of synthesis of 5'-O-substituted 4'-ethynylostavudine derivatives represented by the general **Formula 1,** wherein **R** represents 12-tetradodecanoyl, 12-bromododecanoyl, 12-methoxydodecanoyl, 12-ethylothiododecanoyl group, represented by the symbols **WA37, WA38, WA40, WA42** respectively, comprises treating the compound represented by the Formula 1 (wherein **R** represents hydrogen) and the symbol **WA32** in dichloromethane in the presence of dimethyloaminopyridine at room temperature, for one hour with a respective halogene of above mentioned fatty acid represented by the Formula **RX,** wherein **R** represents.as above, and **X** represents halogen **(Scheme 1).**

5. The method of synthesis of 5'-O-substituted 4'-ethynylostavudine derivative represented by the general **Formula 1**, wherein **R** represents 12-azidododecanoyl group **(WA39)** comprises treating the compound represented by the **Formula 1** wherein **R** represents , 12-bromododecanoyl group **(WA38)** with sodium azide in dimethyloformamide at room temperature **( Scheme 2).**

6. Pharmaceutically acceptable form of drug containing known, pharmaceutically acceptable media and auxiliary substances, containing as an active agent the derivatives represented by the general **Formula 1,** wherein **R** represents 12-tetradodecanoyl, 12-bromododecanoyl, 12-methoxydodecanoyl, 12-ethylothiododecanoyl, and/or 12-azidododecanoyl group according to the claim 1.

7. The form according to the claim 6, containing as an active component a mixture of whichever of the compounds represented by the general **Formula 1,** wherein **R** represents 12-tetradodecanoyl, 12-bromododecanoyl, 12-methoxydodecanoyl, 12-ethylothiododecanoyl, or 12-azidododecanoyl group (the compounds represented by the symbols **WA37, WA38, WA40, WA42, WA39)** with the compound represented by the symbol **WA32.**
